# EUROPEAN PATENT APPLICATION

(11) **EP 3 473 186 A1**
(43) Date of publication of application: **24.04.2019**
(21) Application number: 17196991.8
(22) Date of filing: 18.10.2017
(51) Int. Cl.: A61B 6/08, A61B 6/00

(54) **RADIATION TARGET INDICATION**

(71) Applicant: Koninklijke Philips N.V., 5656 AE Eindhoven (NL)
(72) Inventor: MAACK, Hanns-Ingo, 5656 AE Eindhoven (NL)
(74) Representative: de Haan, Poul Erik

(57) **Abstract**

A system and method for indicating a desired target radiation area of a diagnostic radiation beam on a subject comprising a first and second light field generator to project light fields on a subject. Both light fields are decoupled from path of the diagnostic radiation beam. The overlapping area of both light fields indicates the target radiation area.

## Description

### FIELD OF THE INVENTION

The present invention generally relates to a system and method for indicating a desired target radiation area of a diagnostic or therapeutic radiation beam on a subject and an imaging system for irradiating a subject with a diagnostic or therapeutic radiation beam on a target radiation area on a subject.

### BACKGROUND OF THE INVENTION

Before medical radiography procedures, for instance diagnostic or therapeutic radiography procedures, it is necessary to indicate a desired target area on a subject to be irradiated to align the radiation beam such that it irradiates the relevant sections in the subject (e.g. an organ in a patient) and to avoid irradiating areas that should not be exposed to radiation.

In most radiography procedures target indication is provided using a light field indicator that projects an optical light field on the subject that is shaped such that it corresponds with the area that is to be irradiated with the radiation beam during the procedure. The radiation beam is then collimated such that it corresponds to the indicated target area.

The light field to indicate the desired target area is generated by a light field generator, usually a visible light source producing a light beam. Since the visible light source should not be in the path of the radiation beam, it is usually placed near, but to the side of the radiation source for the procedure. By use of one or more mirrors the light beam is steered to correspond with the path of the radiation beam for the procedure. Therefore the position of the light field generator must virtually correspond with the focal spot, taking the mirror into account.

Usually the light field indicator is integrated in a collimator arrangement for collimating the radiation beam to obtain the same collimation of the light beam of the light field indicator as the radiation beam for the procedure.

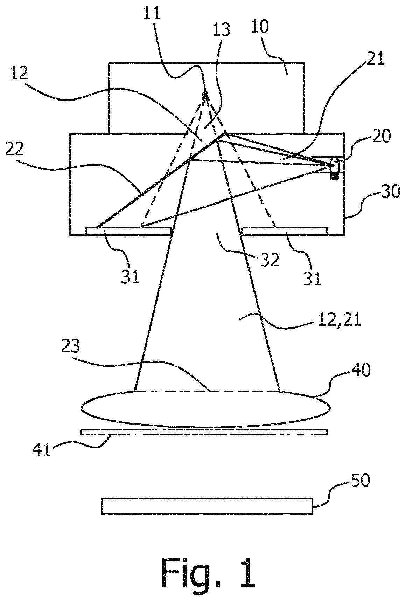

Fig. 1 shows an example of a state of the art light field indicator for an imaging system. A subject 40, e.g. a human or an animal, is placed in an examination area of a medical imager comprising a radiation source 10 and a collimator box 30 comprising a collimator arrangement 31 of collimator blades and a light field generator 20 (usually a lamp emitting light in the visual spectrum). The light field 21 emitted from the light field generator 21 is emitted towards a mirror 22, which reflects the light field 21 towards the subject 40 through a collimator aperture 32. The reflected light field 21 thereby forms a target indication area 23 on the subject 40, which is visible to a technician or physician. The target indication area 23 may be manipulated by moving the collimator blades of the collimator arrangement 31 to form a wider, smaller or differently shaped collimator aperture 32 and corresponding target indicator area 23. When the target indicator area 23 has the correct dimensions then the imaging procedure may start by switching on the radiation source 10. A radiation beam 12 is emitted from focal point 13 through a source aperture 13 and the collimator aperture 32. The mirror 22 should transparent to the wavelength of the diagnostic or therapeutic radiation beam 12. The focal spot 13, source aperture 12 and collimator aperture 32 are aligned such that the radiation beam 12 and the reflected light field 21 correspond and cover the same area 23 on the subject 40.

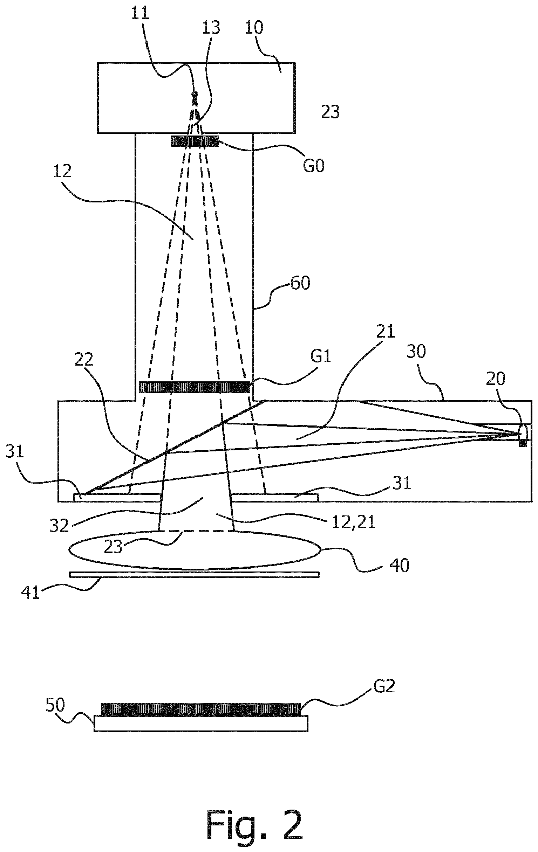

This set-up is suitable for most imaging radiation systems including traditional x-ray imaging and computed tomography imaging. However, for some imaging radiation systems this set-up has disadvantages, particularly for differential phase contrast imaging (DCPI) [see for instance: F. Pfeiffer, C. David & O. Bunk in 'Phase retrieval and differential phase-contrast imaging with low-brilliance X-ray sources', Nature Physics, vol. 2, num. 4, p. 258-261, 2006]. In DCPI at least one optical grating G0 (also known as source grating) is placed between the x-ray radiation source 10 and the subject 40 to be imaged, for instance in a Talbot Lau interferometer set-up, which is shown in Fig. 2. A second grating G1 (also known as phase grating) may be placed in front or behind the subject 40. A third grating G2 (also known as analyzer grating or analyzer absorption grating) is normally placed between the subject 40 and the radiation detector 50. The source grating divides the radiation beam 12 into a plurality of individual coherent beams that are each slightly refracted when they pass through the subject 40. When all dimensions are matched in the correct manner, the resulting deviation in the angle is then determined by the combination of the phase grating G1 and analyzer grating G2 and the local transmitted intensity changes due to the refraction is detected by the radiation detector 50. Both phase contrast and absorption images are obtained simultaneously. In differential phase contrast images much better contrast is achieved between different soft tissue areas compared to absorption imaging with the same high intensity 'hard' x-ray imaging.

While DCPI is a promising new imaging technique, one drawback is that the known light field indicator to define a desired target area, as described previously, is practically not particularly suitable for DCPI imaging.

In practical systems, the distance between the focal spot 11 and the source grating may be around 32 cm. In the often used so-called asymmetric geometry the distance between the source grating G0 and the phase grating G1 is smaller than the distance between the phase grating G1 and the analyzer grating (e.g. G0-G1 = 69 cm and G1-G2 is 150 cm. These distances are not limiting to this invention, for instance also other configurations, e.g. different distance between focal spot 11 and the source grating, or a symmetric arrangement where the distance between the source grating G0 and the phase grating G1 and the distance between the phase grating G1 and the analyzer grating is equal, e.g. 120 cm each. In any case, the distance between the source grating G0 and the phase grating G1 (whether it is in front of the subject 40 as shown in Fig. 2 or behind the subject 40) is normally quite large. This causes the grating housing 60 to extend quite far into the examination area, which might result in insufficient space for the subject 40, particularly when the collimator box 30 with the collimator arrangement 31 and the light field indicator system 20, 22 needs to be added as well, leaving less room for the subject 40 in the examination area. The subject rests against a patient support 41 during an imaging procedure, such as a front cover in imaging systems where the patients stands or a table surface in imaging systems where the subject lies on a table. Visibility linearly increases with distance, so the subject 40 should not be placed too far from the source 10. Because of this the distance between the subject support 41 and the phase grating G1 needs to be rather close, even without a collimator box as shown in Fig. 2. Also there is always some distance (usually about 20 to 40 cm) between the subject support 41 and the analyzer grating G2. This all results in a relatively tight area with limited space for the subject 40. This is a substantial drawback since it would become uncomfortable for the subject 40, being closely sandwiched between the collimator box 30 and the subject support 41.

Furthermore, because the mirror 22 is, in the configuration shown in Fig. 2, positioned much further from source 10 and the radiation beam 12 is much wider at that position, the mirror 22 needs to be much larger. This may result in an even bulkier collimator box 30 and/or increased costs.

Since the light field generator 20 needs to be in the same (virtual) position as the focal spot 11 of the radiation beam 12 this length should be added to the distance of the light field generator 20 to the mirror 22, causing the collimator box 30 to be extended quite far if one would like to use a traditional light field indicator as shown in Fig. 1. A configuration as schematically shown in Fig. 2 is then the result. Such an unwieldy and bulky system is certainly undesired, especially if it would need to be movable. Therefore it would be desirable if there would be an alternative way to indicate a target area for phase contrast imaging and other potential imaging types for which the regular light field indicator system is not or less suitable.

### SUMMARY OF THE INVENTION

Embodiments according to the present invention are directed to a system for indicating a desired target radiation area of a diagnostic or therapeutic radiation beam on a subject. The system comprises a first light field generator that is configured to generate and project a first light field towards the subject in a first light field direction. A second light field generator is configured to generate and project a second light field towards the subject in a second light field direction. The first light field and the second light field at least partially overlap each other on the subject forming an overlapping light field area indicating the desired target radiation area. In other words: the path of the two light fields generated by the light field generators are decoupled from the path of the radiation beam and projected to the subject from a different path. Each generated light field does not follow the same path as the radiation beam, but it has its own path and collimation arrangement, such that the light field does not have to pass through any necessary object to influence the radiation beam of a radiographic procedure. This allows for using light field target indicators in radiology systems for which that is currently not possible or particularly non-advantageous.

In an embodiment the first light field generator is preferably arranged such that a parallax of a generated first light field is larger in the first light field direction than in other directions and the second light field generator is arranged such that a parallax of a generated second light field is larger in the second light field direction than in other directions. In known light field indication systems the parallax needs to be as homogeneous as possible in all directions. This is not a requirement for indicators according to this embodiment of the present invention.

In an embodiment a first collimator arrangement is arranged to collimate the first light field and a second collimator arrangement is arranged to collimate the second light field. As such each light field may be individually collimated dependently or independently of each other.

In an embodiment the first light field generator comprises a first mirror for projecting the first light field towards the subject and/or the second light field generator comprises a second mirror for projecting the second light field towards the subject. Using mirrors allows the light field collimation arrangements to be less bulky or be placed away from already crowded areas of an imaging system.

In an embodiment the first light field generator is adapted to generate the first light field with a first color and the second light field generator is adapted to generate the second light field with a second color, wherein the second color is different from the first color. When both light fields have a different color, the colors will blend when they overlap creating a new color, thereby clearly delineating the desired target area.

Further embodiments of the invention are directed towards a radiology system for irradiating a subject with a diagnostic or therapeutic radiation beam on a target radiation area on the subject. The radiology system comprises a system for indicating the desired target radiation area on the subject according to any of the previously mentioned embodiments of the system for indicating a desired target radiation area. The radiology system further comprises a radiation source arranged to emit radiation beam such that it irradiates the subject on substantially only the indicated target radiation area. In an embodiment the radiation source is an x-ray radiation source and further comprising a flat-panel or curved x-ray radiation detector placed opposite the radiation source across an examination region for receiving the subject. In another embodiment the radiation source is an x-ray or gamma ray source suitable for use in therapeutic radiology procedures.

In an embodiment the radiology system comprises at least one optical grating between the radiation source and the radiation detector, preferably a plurality of successive gratings placed between the radiation source and the radiation detector in a differential phase contrast imaging arrangement, and most preferably in a Talbot Lau interferometer differential phase contrast imaging arrangement. The present invention is particularly interesting for use with phase contrast and dark field imaging, since traditional light field indicators are not suitable for such imaging modalities, while the presently claimed invention opens up the use of light field target indication for these imaging systems.

In an embodiment a third collimator arrangement to collimate the radiation beam arranged such that, when in use, the radiation beam irradiates only the desired target area as indicated by the overlapping light field area formed by the first light field and the second light field generated by the system for indicating the desired target radiation area. As such the radiation beam and the light fields are have their own collimation system to dependently or independently collimate each individual radiation beam or light field.

In an embodiment the first collimator, the second collimator and the third collimator are in the same plane. This allows for direct (mechanical) coupling of the collimators.

In an alternate embodiment at least one of the first collimator, the second collimator and, preferably, the third collimator is not in the same plane with the other two collimator arrangements. In this arrangement the collimators are usually indirectly coupled mechanically or electronically. This arrangement may be preferable in certain radiology systems for space and constructional reasons.

Further embodiment of the present invention are directed towards a method indicating a desired target radiation area of a diagnostic radiation beam on a subject comprising the steps of generating a first light field towards the subject in a first light field direction, generating a second light field towards the subject in a second light field direction, wherein the first light field and second light field are arranged to at least partially overlap each other on the subject, thereby forming an overlapping light field area that indicates the target radiation area.

In an embodiment the method further comprises the step of emitting a radiation beam towards the desired target radiation area on the subject, such that the radiation beam irradiates only the desired target area as indicated by the overlapping light field area formed by the first light field and the second light field indicating the desired target radiation area.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a state of the art light field indicator for a radiation imaging system.
Fig. 2 shows how a state of the art light field indicator must be adapted for use in a phase contrast imaging system.
Fig. 3 shows a schematic top view of a light field indicator system and a collimator arrangement as described in conjunction with the presently claimed invention.
Fig. 4 shows a schematic depiction of a light field indicator system that is in the same plane with the collimator arrangement as described in conjunction with the presently claimed invention.
Fig. 5 shows a schematic depiction of a light field indicator system that is not in the same plane with the collimator arrangement as described in conjunction with the presently claimed invention.
Fig. 6 shows a flow chart of a method for indicating a desired target radiation area as described in conjunction with the presently claimed invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not according to scale.

### DETAILED DESRIPTION OF EMBODIMENTS

The presently claimed invention provides a novel system to indicate a target radiation area that would be suitable for diagnostic or therapeutic radiology systems for which the known target indication method using a light field (as described previously in relation to Fig. 1) is not suitable or provides strong disadvantages. The claimed novel systems and method is illustrated mainly for differential phase contrast imaging (DCPI) system, but a skilled person would know how to adapt the described embodiments to be used in other imaging or therapeutic radiology systems which have the same or similar problems as DCPI systems.

The presently claimed invention is based on the insight that instead of using a single light beam generator used in conjunction with the radiation beam collimator arrangement, the light field can be generated by coupling two light field generators each with a different light beam direction to generate the desired target area.

The term light field generator in the context of the claimed invention means any light source to generate a suitable optical light field, such as a lamp (e.g. incandescent lamps, individual or grouped LEDs) or any other suitable light emitting devices. The light field generator also may include further components, such as components to influence the direction or parallax of the emitted light field.

By using two light field generators problems due to the presence of gratings may be mitigated, as will be explained later. Of course adding a second light field generator increases the number of hardware components, but the end result will still be much less bulky than as shown in for instance Fig. 2, while the generated target indication area is of at least the same quality and preciseness as that of known systems.

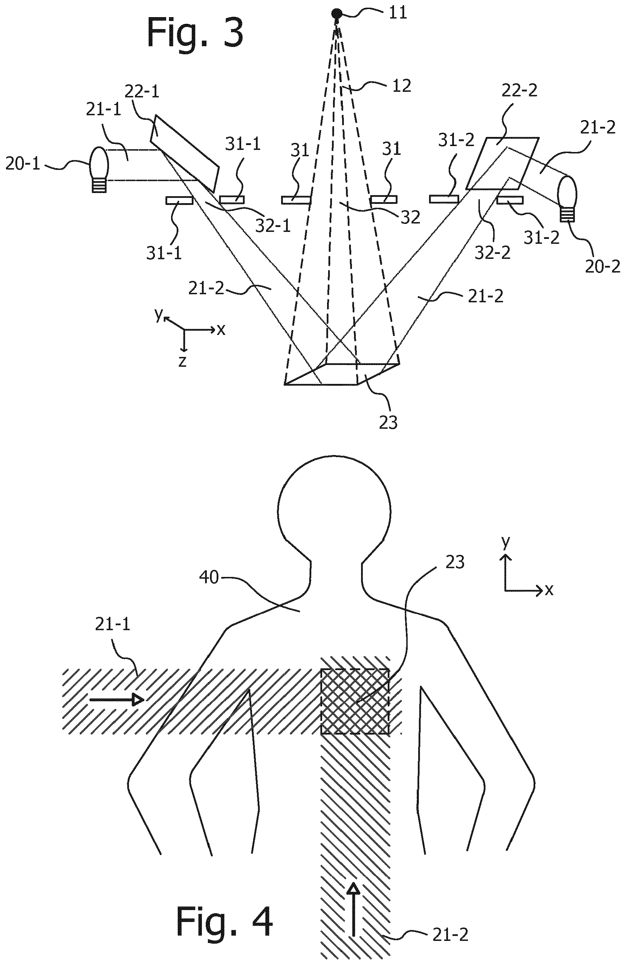

The two light field generators each individually generate a light field, but they are both in a different direction, preferably perpendicularly to each other (e.g. one in the x-direction, the other in the y-direction, as is for instance shown in Fig. 3). The path of the two light fields generated by the light field generators are decoupled from the path of the radiation beam and projected to the subject from a different path, e.g. from the sides of the radiation beam. In other words, each generated light field 21-1, 21-2 is not meant to follow the same path as the radiation beam 12, but it has its own path and collimation arrangement 31-1, 31-2, such that the light field 21-1, 21-2 does not have to pass through be absorbed by) the phase grating G1. In this way the light fields 21-2, 21-2 reaches the subject 40 without absorption by the phase grating G1 to form a clear target indication area 21.

Fig. 3 shows a schematic view of an embodiment of the claimed invention. A first light field generator 20-1 to generate a first light field 21-1 in a first direction x. The first light field 21-1 is reflected by a first mirror 22-1 towards a subject 40.

A second light field generator 20-2 to generate a second light field 21-2 in a second direction y. The second light field 21-2 is reflected by a first mirror 22-1 towards a subject 40.

While the present invention is illustrated using mirrors 22-1, 22-2 to steer the light fields 21-1, 21-2, it would actually be possible, unlike known light field indicators, to construct the light field indication system without any mirrors and directly project the light field from the light field generators 20-1, 20-2 to the subject 40. This may reduce cost of the system, but potentially at the cost of needing a more bulky arrangement.

The first light beam 21-1 and second light beam 21-2 are collimated by respective collimator arrangements 31-1, 21-2 to collimate the light beams 21-1, 21-2 to the desired width.

The positioning of the respective light field generators 20-1, 20-2, the mirrors 22-1, 22-2 and the collimator arrangements 31-1, 31-2 is such that the resulting light beam are directed to the same area on the subject, such that the first light field 21-1 and the second light field 21-2 overlap on the subject 40 to form a clearly marked area 23 of overlapping beams 21-1, 21-2 that indicates a desired target area.

In a particularly advantageous embodiment, the first light field 21-1 has a parallax that is larger in the first direction x than in any other direction, preferably there is no parallax in any other direction than the first direction x. The second light field 21-2 a parallax that is larger in the second direction y than in any other direction, preferably there is no parallax in any other direction than the second direction y. Such an embodiment is in stark contrast with known light field indicators, where the parallax of the generated light field must have as little directional deviation in as possible, because this would cause the projected position to be incorrect. The insight that the parallax actually does not have to be directionally homogenous is the basis for this aspect of the invention. Due to the precision of the geometrical position of the light source, there will be a small parallax which will be larger in one direction than the other. Each light field according to this aspect of the present invention should preferably have a minimal parallax in one direction for the same reasons as for the known light field indicators. However, in contrast to those known indicators, the parallax of the indicators according to this aspect of the present invention in the other direction does not matter and is actually allowed to be quite large. Due to the more unidirectional parallax there is not much stray light in non-relevant directions and the edges of the light beams 21-1, 21-2 on the subject 40 are clearly delimited, especially in the overlapping area, providing a good visual indication of the indicated target area 23 to a physician or radiology specialist.

The resulting first light beam light beam 21-1 and second light beam 21-2 are also shown schematically in Fig. 4 as seen from above and projected onto the subject 40. The first light beam 20-1 is shown in a hatch pattern in one direction, while the second light beam 21-2 is shown in a hatch pattern in a second direction, perpendicular to the first hatch direction to indicate the different parallax of each light beam 21-1, 21-2. In the area 23 where the first light beam 21-1 and the second light beam 21-2 overlap, the section is now indicated by a crosshatch pattern (marked for clarity on the drawing with a square). This area indicates a target area 23 on the subject. Should this area not be of the right size then the collimator arrangements 31-1, 31-2 for the light beams 21-1, 21-2 may be used to enlarge or decrease the beam size in one or both directions x, y. If the target area is not at the right position, the subject 40 may be moved, such that the overlapping light area 23 corresponds to the desired target area 23 as defined by a physician or radiation imaging specialist. Alternatively the light fields 21-1, 21-2 may be moved, e.g. by having movable mirrors 22-1, 22-2 or translatable light field generators 20-1, 21-1. However, this would increase the technical complexity of the system.

The desired target area 23 is already quite well visible on the subject 40 , especially if the light beams 21-1, 21-2 have a unidirectional parallax, but it could be further enhanced when the individual light field generators 20-1, 20-2 each generates a different color light. In the overlapping area the colors would then mix and produce a new color (e.g. the first light field 21-1 is yellow colored, the second light field 21-2 is blue colored, resulting in the overlapping area 23 to be green colored). It would be even easier to find the boundaries of the desired target area 23 in the manner.

Alternatively or additionally, an optical sensor may be integrated in the radiology system that can measure light intensity on different positions on the subject 40, such that it determines where the light beams 21-2 overlap (having a significantly higher brightness than the surroundings). The optical sensor may transmit this information electronically to provide a visualization for the user or to a controller controlling the collimation arrangement 31 for the radiation beam 12, such that the beam may be automatically collimated to the correct size.

The first direction x and the second direction y of the light beams 21-1, 21-2 does not have to be exactly perpendicular to each other. They can actually be in any direction with respect to each as long as they would generate a clearly demarked overlapping area 23 that makes technical sense with respect to imaging a subject with a radiation beam. Also more than two light beam generators could be used. Further, the target radiation area does not have to be square as shown in the Figs, it may also be rectangular, circular, triangular, etc, to fit the shape of the radiation beam imaging the subject 40.

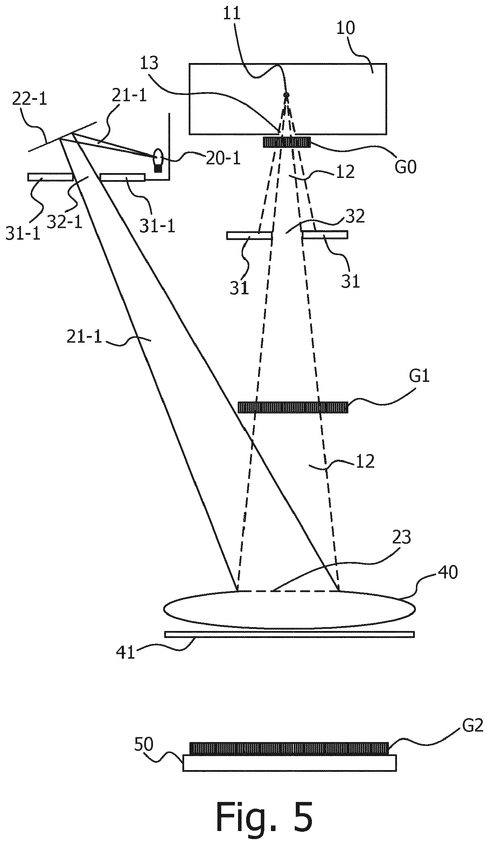

The positioning of the light field generators 20-1, 20-2, mirrors 22-1, 22-2 and collimator arrangements 31-1, 31-2 is not restricted to the embodiment shown in Fig. 3. For instance a 'reverse' set-up in which the light fields 21-2 are initially directed away from the path of the radiation beam 12 and then reflected by mirrors 22-1, 22-2 towards the subject 40. Such a set-up is schematically shown in Fig. 5 (for clarity only the first light field generator 21-1 is shown) is also a very suitable embodiment to implement the invention. Fig. 5 also shows how the subject 40 and how source grating G0, phase grating G1 and analyzer grating G2 could be added in a Talbot-Lau DCPI set-up (not shown in Fig. 3 to avoid clutter in the drawing).

The choice of the arrangement in Fig. 3 or the reverse arrangement in Fig. 5 depends on the available space and dimensions of the imaging system since the two arrangements (and variations thereof) have different housing dimensions and one may be suited better for one type of imaging system design and the other in another design.

When the indicated target are 23 is approved by the physician or radiology specialist then the imaging radiation beam settings may be set such that the radiation beam will only irradiate within the indicated target area 23. For an imaging procedure a radiation beam 12 is emitted from a focal point 13 of a radiation source 10. For imaging systems for which the currently presented target indication system is particular suitable, the radiation source 10 is usually an x-ray radiation source emitting monochromatic or polychromatic x-ray radiation from a single focal spot 11 or from multiple focal spots 11. The presently claimed invention is suitable for all types of x-ray imaging, such as traditional x-ray imaging (e.g. mammography), tomosynthesis or computed tomography imaging. It is particularly suitable for an x-ray imaging system (including computed tomography systems that may utilize a light field target indication system) adapted for DCPI. Usually the radiation beam 12 has a fan-beam shape. The size of the radiation beam 12 is determined by a collimator arrangement 31 which allows a wider or smaller area on the subject 40 present in the examination area to be irradiated by the beam. Behind the examination area a radiation detector 50 is placed to detect radiation traversing through the examination region (and, when present, the subject 40). The detector 50 may be a flat-panel detector or a curved detector.

The presently claimed invention is also suitable for use in radiography systems and potentially also some radiotherapy systems using x-rays, gamma rays and the likes to precisely indicate the radiation target area 23 on the subject 40 to treat tumors and other malignancies treatable by radiotherapy. The presently claimed invention may actually open up the possibility for using a light field target indication system in radiology systems such as for instance radiographic imaging systems using slot-scanning technology or any system in which it is not possible to project a light field to a subject using the exact path of the imaging or therapeutic radiation beam.

The collimator arrangement 31 for the radiation beam 12 must be set such that it collimated the beam as indicated by the target indication area 23 as defined by the first light field 21-1 and the second light field 21-2. The radiation beam collimation should be such that the area irradiated on the subject 40 by the collimated radiation beam 12 preferably is substantially the same as the area 23 as indicated by the overlapping light fields 21-1, 21-2. If the area of radiation beam 12 on the subject 40 is substantially larger than the indicated target area 23 then the subject receives unnecessary radiation on areas not of interest to the imaging procedure If the area of radiation beam 12 on the subject 40 is substantially smaller than the indicated target area 23 then not all of the desired area 23 is imaged and malignancies or other features-of-interest may be missed.

Unlike the known light field indicators the radiation beam 12 and light fields 21-1, 21-2 do not use the same collimator arrangement, instead each has its own collimator arrangement 31, 31-1, 31-2. Therefore the collimation gap 32 of the radiation beam 12 must be determined form the collimation gaps 32-1, 32-2 of the light fields 31-1, 31-2. This may be done by mechanically linking the collimator arrangement 31 for the radiation beam 12 with the collimation systems 21-1, 31-2 for the light beams. This would be the most direct embodiment, but it might be somewhat challenging to design a practically suitable version.

Alternatively the collimation information of the light fields 21-1, 21-2 may be transmitted electronically (wired or wirelessly) to the radiation beam collimator arrangement 31 or to a central controller to control all collimator arrangements 31, 31-1, 31-2. As such, only transmitters and receivers connected to actuators actuating the collimator arrangements 31, 31-1, 31-2 need to be implemented, saving space and avoiding practical complications. As mentioned previously, a light sensor may be integrated in the radiology system to detect the overlapping area of the light beams 21-1, 21-2 and this information may be automatically transmitted to the controller of the radiation beam collimator arrangement 31.

Another option may be to provide a visual indication of the collimation settings of the light field collimator arrangements 31-1, 31-2 for the physician or radiology specialist to manually adapt the collimation gap 32-2 of the radiation beam 12. This would technically be the simplest, but at the cost of accuracy.

The light field collimator arrangements 31-1, 31-2 may be in the same plane as the radiation beam collimator arrangement 31 (as is shown in Fig. 3) or at least one of the collimator arrangements 31, 31-1, 31-2, preferably the radiation beam collimator arrangement 31, is out of plane with the other two collimator arrangements (as is shown in Fig. 5).
A benefit of the in-plane configuration is that the interactive movements of the radiation beam collimator blades 31 could be directly mechanically coupled to those of the light field 31-1, 31-2, so that they open or close with exactly the same distance.

For the out-of-plane configuration the distance of opening or closing the light field collimators 31-1, 31-2 must be scaled with respect to the radiation beam collimator arrangement 31. This may be implemented as a mechanical solution, for instance with a lever-arm. This solution is close to the original manner of adjusting a light field target indication where a mechanical knob for adjusting the collimation is directly coupled to the mechanics. In more modern systems such a knob is usually a potentiometer or other electronic (or software) means that is read oud electronically and controls a motor setting the mechanical position for the collimator arrangement 31. This is easily adaptable to also control separate motors for moving the light field collimator arrangements 31-1, 31-2 in exactly the same distance.

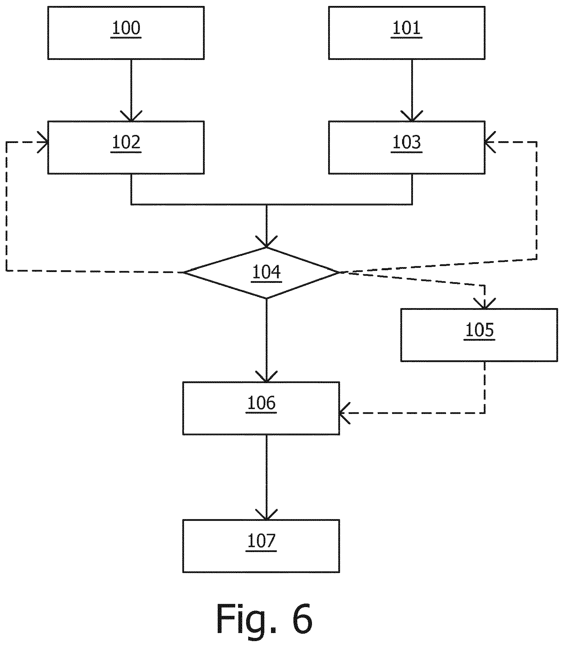

Fig. 6 depicts a schematic flowchart of a method for indicating a desired target radiation area as described in conjunction with the presently claimed invention.

In a first step the first light beam generator is switched on 100 to emit a first light beam in a first direction, preferably with a parallax that is larger in the first direction than in other direction.

After or simultaneously with the first step, the second light beam generator is switched on 101 to emit a second light beam in a second direction, preferably with a parallax that is larger in the second direction than in other direction. The first direction is different from the second direction, preferably substantially perpendicular with respect to each other.

The first light field is collimated 102 with a first collimating system and the second light field is collimated 103 with a second collimating system to adapt the dimensions of the respective light fields. Each light beam may have a different color.

The first and second light field are projected on a subject, usually through respective mirrors in the respective light fields, such that they at least partially overlap each other on the subject forming an overlapping area, indicating a target area for a radiation beam in an imaging procedure.

A physician or radiology specialists checks 104 the indicated area and decides whether the indicated target area is as desired to obtain an optimal imaging area that avoids irradiating non-relevant tissue or omitting part of the area that needs to be imaged. In case the area needs to be enlarged or reduced the collimation of one or both of the light fields may be adapted 102, 103. In case the position of the indicated target area is not deemed to be correct the subject may be repositioned 105 (or alternatively the light beams may be moved 105 if that is possible, e.g. through translating the light field generators or the mirrors). Resizing and repositioning may also both be necessary to be performed, either simultaneously or successively.

When the physician or radiology specialist decides that the indicated target area corresponds to the desired target area, the collimator arrangement of the radiation beam is set 106 to have the radiation beam collimation gap such that the radiation beam fits the indicated target area as precise as is possible and desirable. Next, the imaging procedure may be started 107 by emitting a radiation beam from the radiation source towards the desired target radiation area on the subject, such that the radiation beam irradiates only the desired target area as indicated by the overlapping light field area formed by the first light field and the second light field indicating the desired target radiation area.

While the invention has been illustrated and described in detail in the drawings and foregoing description, such illustration and description are to be considered illustrative or exemplary and not restrictive; the invention is not limited to the disclosed embodiments.

The terms substantially or approximately in the context of this invention means preferably within 10%, more preferably between 5%, even more preferably within 1% and most preferably exactly the indicated value or term.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

Any reference signs in the claims should not be construed as limiting the scope.

## Claims

1. System for indicating a desired target radiation area (23) of a diagnostic or therapeutic radiation beam (12) on a subject (40) comprising:
a first light field generator (20-1), configured to generate and project a first light field (21-1) towards the subject in a first light field direction (x);
a second light field generator (20-2), configured to generate and project a second light field (21-2) towards the subject in a second light field direction (y);
wherein the first light field and the second light field at least partially overlap each other on the subject forming an overlapping light field area (23) indicating the desired target radiation area.

2. System according to claim 1, wherein the first light field generator (20-1) is arranged such that a parallax of a generated first light field (21-1) is larger in the first light field direction (x) than in other directions; and wherein the second light field generator (20-2) is arranged such that a parallax of a generated second light field (21-2) is larger in the second light field direction (y) than in other directions.

3. System according to any of the previous claims, wherein a first collimator arrangement (31-1) is arranged to collimate the first light field (21-1) and a second collimator arrangement (31-2) is arranged to collimate the second light field (21-2).

4. System according to any of the previous claims, wherein the first light field generator (20-1) comprises a first mirror (22-1) for projecting the first light field (21-1) towards the subject (40) and/or the second light field generator (20-2) comprises a second mirror (22-2) for projecting the second light field (21-2) towards the subject.

5. System according to any of the previous claims, wherein first light field generator (20-1) is adapted to generate the first light field (21-1) with a first color and the second light field generator (20-2) is adapted to generate the second light field (21-2) with a second color, wherein the second color is different from the first color.

6. Radiology system for irradiating a subject (40) with a diagnostic or therapeutic radiation beam (12) on a target radiation area (23) on the subject, comprising
a system for indicating the desired target radiation area on the subject according to any of the previous claims;
a radiation source (10) arranged to emit radiation beam 12 such that it irradiates the subject 40 on substantially only the indicated target radiation area.

7. Radiology system according to claim 6, wherein the system for indicating the desired target radiation area on the subject is arranged outside an area covered by the radiation beam when it is switched on.

8. Radiology system according to claim 6 or 7, wherein the radiation source (10) is an x-ray radiation source and further comprising a flat-panel or curved x-ray radiation detector (50) placed opposite the radiation source across an examination region for receiving the subject (40).

9. Radiology system according to any of the claims 6 or 7, wherein the radiation source (10) is an x-ray or gamma ray source suitable for use in therapeutic radiology procedures.

10. Radiology system according to any of the claims 6 to 9, comprising at least one optical grating (G0, G1, G2) between the radiation source (10) and the radiation detector (50), preferably a plurality of successive gratings (G0, G1, G2) placed between the radiation source and the radiation detector in a differential phase contrast imaging arrangement, and most preferably in a Talbot Lau interferometer differential phase contrast imaging arrangement.

11. Radiology system according to any of the claims 7 to 10, comprising a third collimator arrangement (31) to collimate the radiation beam (12) arranged such that, when in use, the radiation beam irradiates only the desired target area (23) as indicated by the overlapping light field area formed by the first light field (21-1) and the second light field (21-2) generated by the system for indicating the desired target radiation area.

12. Imaging system according to claim 11, wherein the first collimator (31-1), the second collimator (31-2) and the third collimator (31) are in the same plane.

13. Imaging system according to claim 11, wherein at least one of the first collimator (31-1), the second collimator (31-2) and, preferably, the third collimator (31) is not in the same plane with the other two collimator arrangements.

14. Method indicating a desired target radiation area (23) of a diagnostic radiation beam (12) on a subject (40) comprising the steps of:
generating (100) a first light field (21-1) towards the subject in a first light field direction (x),
generating (101) a second light field (21-2) towards the subject in a second light field direction (y),
wherein the first light field and second light field are arranged to at least partially overlap each other on the subject, thereby forming an overlapping light field area (23) that indicates the target radiation area.

15. Method according to claim 14, further comprising the steps of
emitting (106) a radiation beam towards the desired target radiation area on the subject, such that the radiation beam irradiates only the desired target area as indicated by the overlapping light field area formed by the first light field and the second light field indicating the desired target radiation area.
